# EUROPÄISCHE PATENTSCHRIFT

(11) **EP 2 373 390 B1**
(45) Veröffentlichungstag und Bekanntmachung des Hinweises auf die Patenterteilung: **05.10.2016**
(21) Anmeldenummer: 09771544.5
(22) Anmeldetag: 04.12.2009
(51) Int. Cl.: B01D 15/00, C12N 15/10, G01N 1/44, G01N 33/50

(54) **PARALLEL-EXTRAKTION VON UNTERSCHIEDLICHEN BIOMOLEKÜLEN AUS FORMALIN-FIXIERTEM GEWEBE**
PARALLEL EXTRACTION OF DIFFERENT BIOMOLECULES MADE OF FORMALIN-FIXED TISSUE
EXTRACTION PARALLÈLE DE DIFFÉRENTES BIOMOLÉCULES DU TISSU FIXÉ AU FORMOL

(30) Priorität: 05.12.2008 EP 08021134
(43) Veröffentlichungstag der Anmeldung: 12.10.2011
(73) Patentinhaber: Qiagen GmbH, 40724 Hilden (DE)
(72) Erfinder: HOLLÄNDER, Vera, 59423 Unna (DE); PORSCHEWSKI, Peter, 40764 Langenfeld (DE)
(74) Vertreter: f & e patent
(86) Internationale Anmeldenummer: PCT/EP2009/066428
(87) Internationale Veröffentlichungsnummer: WO 2010/063837

(56) Entgegenhaltungen:
- WO-A-01/46402
- WO-A-2004/080579
- WO-A-2005/075642
- DE-A1-102005 023 011
- DE-A1-102005 060 738
- "QProteome FFPE Tissue Handbook" [Online] September 2006 (2006-09), QIAGEN , XP002515222 Gefunden im Internet: URL:WWW1.QIAGEN.COM/HB/QPROTEOMEFFPETISSUE KIT_EN> Seite 8 Seite 13 - Seite 15 -& QIAGEN: "Material Safety Data Sheet of Extraction Buffer EXB"[Online] 19. Mai 2008 (2008-05-19), XP002515219 Gefunden im Internet: URL:http://www1.qiagen.com/Support/MSDS/US /Extraction_Buffer_EXB_US2.pdf> [gefunden am 2009-02-11]
- YAMASHITA ET AL: "Heat-induced antigen retrieval: Mechanisms and application to histochemistry" PROGRESS IN HISTOCHEMISTRY AND CYTOCHEMISTRY, GUSTAV FISCHER VERLAG, STUTTGART, DE, Bd. 41, Nr. 3, 29. Dezember 2006 (2006-12-29), Seiten 141-200, XP005818344 ISSN: 0079-6336
- NESATYY VICTOR J ET AL: "Recovery of intact proteins from silver stained gels." THE ANALYST SEP 2002, Bd. 127, Nr. 9, September 2002 (2002-09), Seiten 1180-1187, XP002515220 ISSN: 0003-2654
- BECKER K-F ET AL: "Quantitative protein analysis from formalin-fixed tissues: implications for translational clinical research and nanoscale molecular diagnosis" JOURNAL OF PATHOLOGY, Bd. 211, Nr. 3, Februar 2007 (2007-02), Seiten 370-378, XP002515221 ISSN: 0022-3417

## Beschreibung

Die vorliegende Erfindung betrifft ein Verfahren zur parallelen Isolierung/Extraktion von verschiedenen Biomolekülen, insbesondere Nukleinsäuren und Proteinen aus denselben fixierten biologischen Proben. Die vorliegende Erfindung umfasst auch die Quantifizierung und Analyse der nach dem erfindungsgemäßen Verfahren isolierten Biomoleküle, einen Kit zur parallelen Isolierung/Extraktion verschiedener Biomoleküle aus einer fixierten Probe, sowie die Verwendung dieses Kits für die Diagnose, Prognose, Therapieentscheidung und das Therapiemonitoring einer Krankheit.

Heutzutage ist das Fixieren von Geweben in Formalin zur nachfolgenden histopathologischen Untersuchung Standard, um z. B. gesundes von krankem Gewebe zu unterscheiden. Formalin fixiertes, in Paraffin eingebettetes (FFPE) Gewebe wird seit Jahrzehnten gesammelt und ist die Quelle von unzähligen diagnostischen Studien. Hierbei handelt es sich um Gewebe von den meisten Organen, von verschiedenen Krankheitsstadien, vor/nach/während einer Therapie uvm.. FFPE Gewebe bietet den Vorteil, dass die Morphologie sehr gut erhalten bleibt. Die Formalin-Fixierung von Geweben führt jedoch zu einer starken Quervernetzung von Makromolekülen innerhalb der Zelle, so dass mit bisherigen Ansätzen eine für Klinik oder Forschung stark erwünschte parallele Extraktion von Nukleinsäuren und Proteinen aus einer einzelnen Probe aus menschlichen oder tierischen Geweben nach dieser seit Jahrzehnten bewährten Fixierungsmethode nicht erzielt werden konnte.

Eine parallele Isolierung von verschiedenen Biomolekülen, z. B. von Proteinen und Nukleinsäuren, aus einer einzelnen Probe ist besonders wünschenswert, da zum einen üblicherweise nur eine sehr geringe Menge des Probenmaterials zur Verfügung steht, welches nicht für mehrere getrennte Aufreinigungen ausreichend ist. Zum anderen ist das Probenmaterial heterogen zusammengesetzt, z. B. liegen einige Tumorzellen in einem Verband von gesunden Zellen vor. Ein Trennen oder Teilen der Probe ist in diesem Fall nicht wünschenswert, da bei einer Teilung der Probe nicht garantiert werden kann, dass jede Teilprobe gleichartige Zellen in derselben Menge enthält. Nur die parallele Isolierung der Biomoleküle wie beispielsweise DNA, RNA und Proteine aus einer ungeteilten Probe gewährleistet, dass alle zu untersuchenden Biomoleküle (Analyten) aus einer vergleichbaren Probe stammen und somit zueinander in Bezug gesetzt werden können.

Die Isolierung von Nukleinsäuremolekülen aus FFPE-Geweben ist heutzutage Standard und in der Literatur umfänglich beschrieben. Beispiele für solche Verfahren sind in den internationalen Patentanmeldungen WO2004/080578, WO2005/012523, WO2005/054466 und WO2008/021419 zu finden, wobei diesen Verfahren gemeinsam ist, dass für die Aufreinigung der Nukleinsäuren bevorzugt eine Protease eingesetzt wird, um gleichzeitig vorhandene Proteine, wie bspw. Nukleinsäure abbauende Enzyme zu zerstören.. In solchen Ansätzen ist eine gleichzeitige / parallele Aufreinigung bzw. Isolierung von Proteinen aus derselben Probe selbstverständlich nicht möglich.

Die WO2006/127860 beschreibt die proteolytische Herstellung von Peptidfragmenten aus FFPE-Geweben durch gezielte Proteolyse der in den Geweben enthaltenen Proteine. Hierbei werden zwei Proben von Gewebe gleichzeitig mit unterschiedlichen Puffern behandelt, um ein Peptidmuster aus den Proben zu generieren. Eine gleichzeitige / parallele Protein- und Nukleinsäure-Isolierung aus derselben Probe wird nicht erwähnt.

In der WO2006/122898 wird gezeigt, dass die Extraktion von Proteinen aus fixiertem Gewebe wesentlich verbessert werden kann, wenn die untersuchte Probe zunächst in einem detergenzhaltigen Puffer gekocht und anschließend bei einer Temperatur oberhalb von 60°C weiter inkubiert wird. Eine solche Behandlung der Proben lässt eine anschließende Quantifizierung der isolierten Proteine zu.

In der WO 2007/068764 wird ein Verfahren beschrieben, bei dem eine fixierte Gewebeprobe mit einer vorzugsweise wässrigen Lösung in Kontakt gebracht wird, die ein nukleophiles Reagenz enthält, wodurch die durch die Formalin-Fixierung bewirkte Vernetzung der Biomoleküle leichter gelöst werden kann. In weiteren Aufreinigungsschritten kann die so behandelte Probe entweder zur Isolierung von Proteinen oder zur Isolierung von Nukleinsäuren herangezogen werden. Ein Verfahren zur parallelen / gleichzeitigen Isolierung beider Arten von Biomolekülen aus derselben Probe wird in dieser Anmeldung nicht beschrieben.

Aufgabe der vorliegenden Erfindung war es ein Verfahren bereitzustellen, das es ermöglicht, verschiedene Biomoleküle aus einer einzelnen Probe eines fixierten Gewebes zu erfassen und ggf. zu untersuchen.

Diese Aufgabe wird gelöst durch ein Verfahren zur parallelen Aufreinigung verschiedener Arten von Biomolekülen aus demselben durch Vernetzung fixierten biologischen Ausgangsmaterial, wobei
a) ein Schritt zum Lösen der Vernetzungen des Ausgangsmaterials unter Verwendung eines Puffer, welcher ein Detergenz und keine proteolytisch wirksame Verbindung enthält
b) ein Schritt zur Trennung der in dem Ausgangsmaterial befindlichen unterschiedlichen Biomoleküle in wenigstens eine Fraktion (A), die weitgehend intakte Proteine enthält, und wenigstens eine unlösliche Fraktion (B) und
c) Isolierung oder Nachweis/Analyse oder Isolierung und Nachweis/Analyse unterschiedlicher Biomoleküle aus wenigstens einer der Fraktionen (A) und(B) aus Schritt b)
   erfolgt,
sowie einen Kit zur Durchführung des Verfahrens. Bevorzugte Ausführungsformen sind in den Unteransprüchen enthalten.

Es hat sich überraschend herausgestellt, dass bei der Durchführung bekannter Verfahren, insbesondere bei dem in WO 2006/122898 beschriebenen Verfahren unter (a) Einsatz eines Protease-freien Puffers, (b) Verwendung eines Detergenz und (c) Kochen der Probe und anschließender Inkubation bei oberhalb von 60°C für länger als 50 Sekunden die durch Formalin bewirkte Vernetzung im Gewebe nur bei Proteinen zur ausreichenden Auflösung der Quervernetzungen führt. Daher wird eine weitgehend quantitative Isolierung in bisher beschriebenen Verfahren nur für Proteine erzielt, während die auch in der Probe enthaltenen Nukleinsäuren nicht bzw. nicht in größerem Umfang freigesetzt werden, sondern in der ungelösten Fraktion der Probe verbleiben. Dies führte zu der Erkenntnis, dass sich diese Nukleinsäuren durch einen einfachen mechanischen Abtrennungsschritt, z. B. durch Zentrifugation oder Filtration, von der löslichen Proteinfraktion im wesentlichen abtrennen lassen. Somit ist erfindungsgemäß aus derselben Ausgangsprobe der "Proteinstatus" und der "Nukleinsäurestatus" getrennt voneinander erfassbar. Die Nukleinsäuren können dann aus der ungelösten, abgetrennten Fraktion durch bereits bekannte Nukleinsäure-Isolierungsmethoden, wie z. B: RNeasy FFPE und QIAamp FFPE (beide von Qiagen, Hilden, Deutschland), die eine Protease-Behandlung zum weiteren Aufschluß der ungelösten Fraktion und einen weiteren Hitzeinkubatiosschritt einschließen, isoliert werden. Auf diese Weise werden beide Biomolekülarten aus ein und derselben Probe in einem Extraktionsschritt vorfraktioniert und danach getrennt voneinander isoliert und so für weitere (nachfolgende) Analysemethoden zugänglich gemacht.

Der Begriff Biomoleküle umfasst alle dem Fachmann bekannten Biomoleküle, beispielsweise natürliche oder synthetische, z. B. in die Probe eingebrachte Nukleisäuren, lineare, verzweigte oder zirkuläre, einzel- oder doppelsträngige Nukleinsäuren, RNA, insbesondere mRNA, siRNA, miRNA, snRNA, tRNA, hnRNA oder Ribozyme, genomische, plasmidäre oder Organellen-DNA, Proteine, Peptide und modifizierte Proteine sowie Nukleinsäuren, Proteine und Peptide infektiösen Ursprungs, Antikörper, Hormone, Wachstumsfaktoren, Lipide, Oligosaccharide, Polysaccharide, Proteoglukane, Metaboliten und Wirkstoffe/Medikamente ohne hierauf beschränkt zu sein.

Als biologische Probe kommen alle zur Fixierung geeigneten biologischen Proben in Betracht, wie beispielsweise zellhaltige Körperflüssigkeiten wie Blut, Sperma, Cerebrospinalflüssigkeit, Speichel, Sputum oder Urin, Leukozyten-Fraktionen, "buffy coat", Fäkalien, Abstriche, Punktate, Hautfragmente, ganze Organismen oder Teile daraus, Organe, Organfragmente, Gewebe und Gewebeteile von Mehrzellern, vorzugsweise von Insekten und Säugetieren, insbesondere vom Menschen, beispielsweise in Form von Sektionen, Biopsien, Fine-Needle-Aspirates oder Gewebeschnitten, isolierte Zellen, beispielsweise in Form adhärenter oder suspendierter Zellkulturen, Pflanzen, Pflanzenteile, Pflanzengewebe oder Pflanzenzellen, Bakterien, Viren, Hefen und Pilze, ohne auf diese beschränkt zu sein. Als besonders bevorzugtes Ausgangsmaterial kann ein Gewebeschnitt aus menschlichem Gewebe verwendet werden.

Die Fixierung der biologischen Probe kann dabei mit allen dem Fachmann bekannten Fixativen erfolgen, insbesondere mit Säuren, Alkoholen, Aldehyden, Ketonen oder anderen organischen Substanzen, wie insbesondere Glutaraldehyd oder Formaldehyd, wobei mit Formaldehyd fixierte biologische Proben besonders bevorzugt sind. Gemäß einer besonders bevorzugten Ausführungsform des erfindungsgemäßen Verfahrens wird eine mit Formaldehyd fixierte und in Paraffin eingebettete biologische Probe verwendet.

Formalin fixierte, Paraffin eingebettete (FFPE) Gewebe werden weltweit routinemäßig zur histopathologischen Diagnose von erkranktem und gesundem Gewebe genutzt, weil in FFPE Gewebe die Morphologie sehr gut erhalten bleibt. Makromoleküle wie DNA, RNA und Proteine können in derart fixiertem Gewebe allerdings meist nicht mehr adäquat untersucht werden. Entgegen langjährigen Erfahrungen von Wissenschaftlern wurde jüngst eine Methode zur Extraktion von intakten Proteinen aus FFPE Gewebe etabliert: Becker et al beschrieben eine exakte Bestimmung des Expressionsgehaltes von Proteinen aus klinischen Gewebsproben *(*J Pathol. 211:370-8, 2007*).* Klinischen Forschern wurde damit die Möglichkeit zur quantitativen Proteinanalyse an FFPE Geweben und der Vergleich mit vorhandenen klinischen Daten eröffnet.

Analysemethoden bzw. Nachweismethoden für die nach dem erfindungsgemäßen Verfahren isolierten Nukleinsäuren und Proteine können alle dem Fachmann bekannten Analysemethoden sein, wie beispielsweise Amplifikationstechniken wie z. B., PCR, qPCR, RT-PCR, qRT-PCR, Whole Genome Amplification, Gelelektroporese, Blotting-Technologien, wie Southern-Blotting, Northern-Blotting und immunologische Verfahren wie Western-Blotting, die Immunpräzipitation oder die Affinitätschromatographie, Mircoarray-Analysen, RFLP-Analyse (restriction fragment length polymorphism-Analyse), SAGE (serielle Analyse der Genexpression), Sequenzierung, SNP-Analysen, Mutationsanalysen, epigenetische Analysen wie z. B. Analyse von Methylierungsmustern, Protein-/Antikörperarray, Immunpräzipitation, High Performance Liquid Chromatography (HPLC), Fast Protein Liquid Chromatography (FPLC),SELDI- oder SELDI-TOF, Massenspektroskopie, MALDI-TOF-Massenspektrometrie, Enzyme-Iinked Immonosorbent-Assays (ELISA), PolyacrylamidGelelektrophorese (PAGE), insbesondere zweidimensionale PolyacrylamidGelelektrophorese (2-D-Gelelektrophorese), Kapillarelektrophorese, bei Enzymen auch der Nachweis über deren spezifische Aktivität und dergleichen mehr, ohne auf diese beschränkt zu sein.

Die vorliegende Erfindung beschreibt eine Methode zur parallelen Isolierung von Biomolekülen, bevorzugt von Proteinen und Nukleinsäuren, wie beispielsweise genomischer (g)DNA oder RNA aus FFPE Gewebe und deren Analyse mittels sensitiver und quantitativer Methoden, wobei von sehr geringen Probenmengen wie z.B. aus klinischen Biopsien ausgegangen werden kann.

Unter paralleler Isolierung gemäß der vorliegenden Erfindung ist zu verstehen, dass ausgehend von derselben Probe (d.h. aus dem identischen Ausgangsmaterial) verschiedene Arten von Biomolekülen, beispielsweise sowohl Nukleinsäuren als auch Proteine jeweils aufgereinigt werden. Hierzu werden beispielsweise die Proteinfraktion und die Nukleinsäurefraktion nach einem ersten Schritt des Lösens der durch Formalin bewirkten Vernetzungen voneinander getrennt und aus wenigstens einer dieser Fraktionen, bevorzugt aus jeder Fraktion, die Biomoleküle, bevorzugt die in der jeweiligen Fraktion überwiegend vorkommenden Biomoleküle isoliert. Bevorzugt werden aus der löslichen Fraktion die Proteine isoliert, aus der unlöslichen Fraktion die Nukleinsäuren. Diese weitere Isolierung nach der Trennung der Biomoleküle aus den Fraktionen kann gleichzeitig oder nacheinander erfolgen.

Durch das erfindungsgemäße Isolierungsverfahren können sowohl Nukleinsäuren, wie auch Proteine aus derselben Ausgangsprobe weitgehend quantitativ voneinander getrennt isoliert werden. Durch dieses Verfahren ist es beispielsweise auch möglich die Struktur, Sequenz und den Methylierungszustand von Genen und/oder die Expression von Genen und mit dem in dem Gewebe auftretenden Proteinstatus zu vergleichen.

Das hier beschriebene Verfahren kann z.B. eingesetzt werden, um diagnostisch oder therapeutisch wichtige Marker *in vivo* im Kontext der komplexen Architektur und Regulationsmechanismen eines betrachteten Gewebes zu erfassen. Um nur ein Beispiel für einen solchen wichtigen Marker zu nennen, sei beispielsweise der epidermale Wachstumsfaktor-Rezeptor (EGFR) in seiner Proteinexpression und seinem DNA-Mutationsprofil in Tumorgewebe genannt.

Die vorliegende Methode kann sowohl für die klinische Forschung als auch für grundlagenorientierte Untersuchungen eingesetzt werden. Noch wichtiger ist, dass das hier beschriebene Verfahren für die Isolierung von Biomolekülen aus Formalin fixierten Geweben optimal in den klinischen Alltag eingebettet werden kann. So können wesentlich genauere Diagnose und Therapieverfahren angewendet werden.

Die hier beschriebene Erfindung betrifft eine wesentliche Optimierung der Extraktion von sowohl Proteinen als auch Nukleinsäuren aus fixierten biologischen Proben wie Gewebeproben zur nachfolgenden Analyse und insbesondere zur Quantifizierung der Proteine und der Nukleinsäuren, die zu aktuellen Hochdurchsatzmethoden, beispielsweise Proteinarrays und real time PCR Verfahren, in der Klinik und in der experimentellen Forschung kompatibel ist. Beispielsweise sind Proben aus verschiedenen Krankheitsstadien oder -verläufen auf diese Weise für die Analyse und Quantifizierung der enthaltenen Biomoleküle zugänglich und erlauben somit auch retrospektive Analysen.

Mit dem vorliegenden Verfahren können intakte Proteine und Nukleinsäuren aus demselben Ausgangsmaterial nachgewiesen und quantifiziert werden. Proteine aus völlig verschiedenen Zellkompartimenten, wie z.B. dem Zellkern, dem Zytoplasma oder der Zellmembran, können sicher isoliert und mengenmässig bestimmt werden. Die isolierten intakten Proteine sind verdünnbar, d.h. es können Verdünnungsreihen und damit interne Standardkurven erstellt werden. Damit kann sichergestellt werden, dass der Nachweis und die Quantifizierung der Proteine im linearen Bereich stattfindet. Die Proteine können vorher bei Bedarf mittels Western blot untersucht werden, um sicher zu stellen, dass keine Kreuzreaktionen der verwendeten Nachweismittel, z.B. Antikörper, auftreten (nur eine spezifische Bande in der richtigen Größe im Western blot). Die auf die hier beschriebene Weise isolierten und quantifizierbaren intakten Proteine ergänzen in optimaler Weise Ergebnisse von immunhistochemischen Analysen, wie sie bereits im klinischen Alltag durchgeführt werden. Damit ist die exakte und sensitive Quantifizierung von intakten Proteinen und die zelluläre Zuordnung von Proteinen (Immunhistochemie) im fixierten Gewebe möglich. Gleichzeitig kann der erfasste Proteinstatus mit dem in dem Gewebe auftretenden Expressionsmuster, oder auch mit der Quantität einzelner exprimierter Gene verglichen werden. Bekannte Krankheitsmarker, beispielsweise Her2/neu bei Brutkrebspatientinnen, können mit hoher Genauigkeit sowohl auf Expressions- wie auch auf Translationsebene klinisch bestimmt werden. Darüber hinaus können neue Krankheitsmarker vergleichend zwischen einem gesunden und einem kranken Gewebe durch die Methode identifiziert werden, indem die exprimierten Gene und /oder die isolierten intakten Proteine mit gängigen Nachweismethoden, z.B. Massenspektroskopie oder quantitativer PCR, analysiert werden. Tierische Gewebe können mit der vorliegenden Methode ebenfalls untersucht werden. Für viele Krankheiten des Menschen, z.B. Tumorerkrankungen, sind bereits Tiermodelle verfügbar. Die zu untersuchenden Gewebe werden typischerweise in Formalin fixiert, in Paraffin eingebettet und dann histopathologisch begutachtet. Die vorliegende Methode kann für eine exakte, sensitive und leistungsfähige Quantifizierung, z.B. mittels Proteinarrays und quantitativer PCR, bekannter und neuer Krankheitsmarker in diesen Modellen eingesetzt werden.

Gemäß dem vorliegenden Verfahren werden die im Gewebe enthaltenen Proteine in einem frühen Verfahrensschritt von den im Gewebe enthaltenen Nukleinsäuren weitgehend quantitativ getrennt.

Unter dem Begriff "weitgehend quantitativ" ist gemäß dem vorliegenden Verfahren zu verstehen, dass nach dem Trennungsschritt zwei verschiedene Fraktionen erhalten werden, die jeweils ursprünglich im Gewebe enthaltene Biomoleküle enthalten, wobei in einer Fraktion (A) mehr als 50% der gesamten löslichen (d.h. der insgesamt aus dem Gewebe herauslösbaren) Proteine enthalten sind, aber weniger als 50% der insgesamt herauslösbaren Nukleinsäuren, während in der anderen Fraktion (B) nach dem Trennungsschritt weniger als 50 % der löslichen Proteine verbleiben, aber mehr als 50 % der Nukleinsäuren. Bevorzugt enthält Fraktion (A) nach dem Trennungsschritt mehr als 60%, weiter bevorzugt mehr als 70%, besonders bevorzugt mehr als 75%, insbesondere bevorzugt wenigstens 80% der insgesamt aus dem Gewebe herauslösbaren Proteine, währen Fraktion (B) mehr als 60%, weiter bevorzugt mehr als 70%, besonders bevorzugt mehr als 75%, insbesondere bevorzugt wenigstens 80% der insgesamt aus dem Gewebe herauslösbaren Nukleinsäuren enthält, wobei diese Nukleinsäuren nach dem ersten Trennungsschritt noch in den unlöslichen Bestandteilen des Gewebes gebunden sein können.

Unter dem Begriff Isolierung soll gemäß der vorliegenden Erfindung das Abtrennen der betrachteten Biomoleküle von sonstigen Bestandteilen des Ausgangsmaterials verstanden werden, ohne dass dabei eine vollständige oder weitgehende Reinigung der Biomoleküle stattfinden muss. Unter einem isolierten Biomolekül ist somit ein Biomolekül zu verstehen, das sich nicht mehr in seiner ursprünglichen, natürlichen Umgebung befindet, bspw. in einer Zelle, sondern aus dieser herausgelöst wurde und von dem andere Zellbestandteile wenigstens teilweise entfernt wurden. So stellt beispielsweise das Trennen von löslichen von unlöslichen Bestandteilen einer Zelle durch Aufschließen der Zelle und anschließende Zentrifugation einen Schritt zur Isolierung der löslichen Zellbestandteile dar.

Unter dem Begriff Aufreinigung ist zu verstehen, dass ein bereits von weiteren Zellbestandteilen getrenntes (isoliertes) Biomolekül wenigstens einem weiteren Reinigungsschritt unterzogen wird, um Verunreinigungen wenigstens teilweise zu entfernen. Beispielsweise können lösliche Proteine durch chromatographische Methoden oder auch durch Dialyse von sonstigen löslichen Zellbestandteilen gereinigt werden. Hierbei ist Reinigung oder Aufreinigung nicht so zu verstehen, dass das gereinigte Biomolekül anschließend ohne irgendeine Art von Verunreinigung vorliegt. Vielmehr wird das Biomolekül bereits dann als gereinigt bezeichnet, wenn das Verhältnis des Biomoleküls zu sonstigen Verunreinigungen durch den Reinigungsschritt zunimmt.

Mit dem Begriff "herauslösbar" oder "lösbar" ist gemeint, dass ein Biomolekül durch ein geeignetes Verfahren isolierbar ist. Der Begriff kann sowohl in Bezug auf die Arten von Biomolekülen (z.B. Proteine oder Nukleinsäuren) wie auch auf die Menge der Biomoleküle, die aus dem Ausgangsmaterial durch geeignete Behandlung isolierbar sind, verwendet werden. Hierbei ist zu beachten, dass ein Biomolekül auch dann als herauslösbar bezeichnet werden kann, wenn es noch im Verband vorliegt, also noch nicht gelöst ist, aber durch ein geeignetes Verfahren gelöst werden kann (beispielsweise auch Membranproteine).

Die einzelnen Schritte des erfindungsgemäßen Verfahrens werden nachfolgend näher beschrieben:
Als Ausgangsmaterial kann jedes Biomoleküle enthaltende Material verwendet werden, bevorzugt wird ein fixiertes Ausgangsmaterial verwendet. Besonders bevorzugt wird ein mit Formalin fixiertes Gewebe, insbesondere ein FFPE-Gewebe verwendet. Wird ein FFPE Gewebe verwendet, wird dies bevorzugt zunächst von Paraffin befreit. Die zu untersuchenden Gewebeareale können entweder manuell als Mikrotomschnitte oder Gewebestanzen oder mittels Laser-Mikrodissektion vom Gewebeschnitt abgenommen werden.

Die zunächst bevorzugt stattfindende Entparaffinierung kann einen deutlichen Einfluss auf die erzielbare Ausbeute an Biomolekülen haben. Die Entparaffinierung dient generell der Entfernung des zur Einbettung der biologischen Probe verwendeten Paraffins. Dieses Paraffin kann im Allgemeinen störend einerseits während des Lösens und der Fraktionierung der Biomoleküle und andererseits während der weiteren Aufreinigung und Analyse der Biomoleküle wirken. Üblicherweise werden Proben durch eine Inkubation in organischen Lösemitteln, wie beispielsweise Xylol und/oder Ethanol entparaffiniert. Dabei wird die Probe in der Regel zunächst ein- oder mehrfach in Xylol inkubiert und dann das Xylol durch eine oder mehrere Inkubationen in 100%igen Ethanol und ggfs. im Anschluß in verdünnnten Ethanol entfernt. Alternativ können auch andere organische Lösemittel zur Entparaffinierung verwendet werden, wie z. B. Alkane, bes. bevorzugt Heptan, oder andere Alkohole, z. B. Methanol, Als besonders vorteilhaft für hohe Ausbeuten qualitativ hochwertiger Biomoleküle nach dem beschriebenen Verfahren hat sich eine Entparaffinierung basierend auf einer Inkubation in Alkanen, wie Heptan, und einem Zusatz von Alkohol, besonders bevorzugt Methanol, erwiesen.

Gemäß Schritt a) des erfindungsgemäßen Verfahrens werden zunächst die in dem Ausgangsmaterial befindlichen Vernetzungen, insbesondere die Vernetzungen der Biomoleküle wenigstens teilweise gelöst.

In einer geeigneten Ausführungsform wird hierfür das Ausgangsmaterial, ggf. nach einem Entparaffinierungsschritt, (i) in ein bevorzugt wässriges System überführt, das ggf. vorteilhafterweise ein Detergenz enthält und frei von proteolytisch wirksamen Verbindungen ist.. Es sollten beispielsweise keine Proteasen, wie Trypsin oder Proteinase K, verwendet werden, um die Intaktheit der Proteine zu gewährleisten. Beispielsweise kann eine wässrige Lösung oder ein Puffer verwendet werden, die/der die weiter unten als vorteilhaft beschriebenen Substanzen enthält.

Das in der Lösung, bspw. in dem Puffer befindliche Material wird (ii) auf eine Temperatur erwärmt, die ausreicht, um die darin befindlichen herauslösbaren

Proteine freizusetzen, bevorzugt wird das Material zunächst gekocht (auf 95°C bis 100°C erhitzt). Die Inkubationszeit kann variieren, von beispielsweise 5 Minuten bis 40 Minuten. Die anzusetzende Zeit des Kochens kann beispielsweise von der Probengrösse abhängen.

Anschließend werden (iii) die Proben bei einer Temperatur oberhalb von 60°C (z.B. 80°C) inkubiert. Die Dauer der Inkubation bei >60°C kann variieren, von beispielsweise 1 Stunde bis 6 Stunden. Die Inkubationszeit bei wärmer als 60°C sollte bevorzugt wenigstens 20 min, jedoch bevorzugt weniger als 16 Stunden betragen. Intakte Proteine werden dadurch in ausreichender Menge in Lösung gebracht und können entweder unmittelbar nachgewiesen, analysiert und/oder beispielsweise quantifiziert werden.

In einer bevorzugten Ausführungsform des erfindungsgemäßen Verfahrens kann das in Schritt (i) eingesetzte wässrige System eine wässrige Lösung, insbesondere ein Puffer sein.

Ein im Sinne der Erfindung geeigneter Puffer bzw. ein Puffersystem weist üblicherweise einen spezifischen pH-Wert im Bereich von 1,0 bis 12,0 auf, bevorzugt zwischen pH 1,0 und 9,0.

Bevorzugt kann das wässrige System wenigstens ein reduzierendes Reagenz, bevorzugt Dithiothreitol (DTT) enthalten. Es wurde festgestellt, dass die Verwendung eines Reduktionsmittels, insbesondere die Verwendung von DTT zu einer besonders vorteilhaften Ausbeute an isoliertem, intaktem Protein führt. Der Vorteil dieser Ausführungsform besteht insbesondere darin, dass die erhaltenen Lysate besonders gut direkt mit kommerziell verfügbaren und dem Fachmann bekannten Proteinquantifizierungsassays wie bspw. BioRad DC® oder BCA-Assay® von Pierce quantifiziert werden können und in den weiteren Analysen eingesetzt werden können. Auf ein Verdünnen der Proben kann verzichtet werden. Dadurch können Messungenauigkeiten vermieden und es kann gewährleistet werden, dass in den nachfolgenden Analysen (z.B. Western Blot) gleiche Proteinmengen eingesetzt werden.

Ferner kann der Puffer zusätzlich reduzierende Reagenzien wie 1 ,4- Dithio-DLthreit, Dithioerythritol (DTE), Tris(2-carboxyethyl)phosphin (TCEP) oder Monoethanolamin (MEA) umfassen.

Die reduzierenden Reagenzien werden in einer Konzentration im Bereich von 0,05 mM bis 20 mM eingesetzt, bevorzugt von 0,1 bis 10 mM, weiter bevorzugt von 0,5 bis 5 mM, besonders bevorzugt von 0,5 bis 2 mM. Die geeignetste Konzentration innerhalb dieses Bereiches hängt im Einzelfall von dem/den eingesetzten Reagenz(ien) ab, beispielsweise ist eine besonders geeignete Konzentration für DTT 1mM.

Detergenzien für das erfindungsgemäße Verfahren können alle dem Fachmann bekannten und zur Zelllyse geeigneten Detergenzien sein, insbesondere werden als Detergenz anionische oder nichtionische Detergenzien verwendet, bevorzugt sulfatgruppenhaltige Detergenzien, besonders bevorzugt Natriumdodecylsulfat

(SDS), Natrium-Deoxycholat, 3-[N-(3-Cholanamidopropyl)-dimethylammonio]-2-hydroxy-1-propansulfonat (CHAPS), Triton X100, Nonidet P40 oder Tween20.

Die Konzentration an Detergenz kann beispielsweise etwa 0.1 -10% sein. Besonders bevorzugt liegt der Konzentrationsbereich im Bereich von etwa 1 -5%.

Bevorzugt kann das wässrige System außerdem wenigstens ein nukleophiles Reagenz enthalten. Als nukleophiles Reagenz sind dabei alle Lewis-Basen geeignet, die in der Lage sind, Elektronen in ein leeres Orbital bzw. in leere Orbitale einer Lewis-Säure zu transferieren. Unter diesen Lewis-Basen besonders bevorzugt sind Reagenzien, welche mindestens eine funktionelle Gruppe aufweisen, die eine negative Ladung trägt, die negativ polarisiert ist oder die mindestens ein freies Elektronenpaar aufweist.

Verbindung umfassend eine funktionelle Gruppe mit einer negativen Ladung sind beispielsweise Alkali- oder Erdalkalialkoxide, Alkali- oder Erdalkalihydroxide, Alkali- oder Erdalkalihalogenide, Alkali- oder Erdalkalicyanide und dergleichen.

Reagenzien, welche mindestens eine funktionelle Gruppe aufweisen, die negativ polarisiert ist, sind insbesondere solche Reagenzien, die mindestens eine funktionelle Gruppe aufweisen, in der zwei kovalent miteinander verbundene Atome vorliegen, die sich in ihrer Elektronegativität nach Alfred und Rochow um mindestens 0,25, besonders bevorzugt um mindestens 0,5 und darüber hinaus bevorzugt um mindestens 1,0 unterscheiden.

Erfindungsgemäss besonders bevorzugte nukleophile Reagenzien sind jedoch solche, die mindestens eine funktionelle Gruppe mit einem oder zwei, besonders bevorzugt mit einem freien Elektronenpaar aufweisen, wobei unter diesen Verbindungen wiederum solche am meisten bevorzugt sind, die mindestes eine primäre, sekundäre oder tertiäre Amino-Gruppe der Struktur I 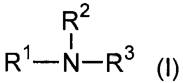 aufweisen, in der R¹ eine C₁- bis C₂₀-Kohlenwasserstoff-Gruppe, bevorzugt eine C₂-bis C₁₅-Kohlenwasserstoff-Gruppe und besonders bevorzugt eine C₂ bis C₁₀-Kohlenwasserstoffgruppe, eine mindestens ein Heteroatom aufweisende C₁- bis C₂₀-Kohlenwasserstoff-Gruppe, bevorzugt eine mindestens ein Heteroatom aufweisende C₂- bis C₁₅-Kohlenwasserstoff-Gruppe und besonders bevorzugt eine mindestens ein Heteroatom aufweisende C₂- bis C₁₀-Kohlenwasserstoff-Gruppe oder ein gegebenenfalls Heteroatom substituiertes aromatisches Ringsystem ist, R² eine C₁-bis C₂₀-Alkylgruppe, bevorzugt eine C₁- bis C₁₀-Alkylgruppe und besonders bevorzugt eine C₁- bis C₂-Alkylgruppe, insbesondere eine Methyl-Gruppe oder eine Ethyl-Gruppe, eine C₁- bis C₂₀-Hydroxyalkylgruppe, bevorzugt eine C₁- bis C₁₀-Hydroxyalkylgruppe und besonders bevorzugt eine C₁- bis C₂-Hydroxyalkyl-Gruppe oder ein Wasserstoffatom ist, wobei ein Wasserstoffatom am meisten bevorzugt ist, und R³ eine C₁- bis C₂₀-Alkylgruppe, bevorzugt eine C₁- bis C₁₀-Alkylgruppe und besonders bevorzugt eine C₁- bis C₂-Alkylgruppe, insbesondere eine Methyl-Gruppe oder eine Ethyl-Gruppe, eine C₁- bis C₂₀-Hydroxyalkylgruppe, bevorzugt eine C₁- bis C₁₀-Hydroxyalkylgruppe und besonders bevorzugt eine C₁- bis C₂-Hydroxyalkyl-Gruppe oder ein Wasserstoffatom ist, wobei ein Wasserstoffatom am meisten bevorzugt ist.

Erfindungsgemäss besonders bevorzugte nukleophile Reagenzien mit einer funktionellen Gruppe der vorstehend dargestellten Struktur I sind insbesondere diejenigen, welche mindestens eine funktionelle Gruppe der Struktur I aufweisen, in der mindestens einer der Reste R² und R³, am meisten bevorzugt beide Reste R² und R³ ein Wasserstoffatom ist bzw. sind. Darüber hinaus sind insbesondere solche nukleophilen Reagenzien bevorzugt, die mindestens eine funktionelle Gruppe der Struktur I aufweisen, in der das Stickstoffatom nur mit solchen Atomen in den Resten R¹, R² und R³ kovalent verknüpft ist, die sp³-hybridisiert sind. Insbesondere sollte keiner der Reste R¹, R² oder R³ in der Lage sein, das freie Elektronenpaar am Stickstoffatom über die Reste R¹, R² bzw. R³ hinweg zu delokalisieren. Somit ist es insbesondere bevorzugt, dass keiner der Reste R¹, R² und R³ beispielsweise die Struktur II 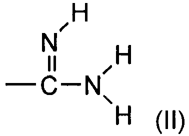 aufweist.

Erfindungsgemäss besonders bevorzugte nukleophile Reagenzien mit mindestens einer funktionellen Gruppe der Struktur I sind ausgewählt aus der Gruppe bestehend aus Methylamin, Ethylamin, Ethanolamin, n-Propylamin, n-Butylamin, iso-Butylamin, tert-Butylamin, Dimethylamin, Diethylamin, Diethanolamin, di-n-Propylamin, di-isoPropylamin, Dibutylamin, Trimethylamin, Triethylamin, Triethanolamin, Hexamethylenetetramin, 2-Ethylhexylamin, 2-Amino-1,3-propandiol, Hexylamin, Cyclohexylamin, 1,2-di-Methoxypropanamin, 1-Aminopentan, 2-Methyloxypropylamin, Tri(hydroxymethyl)-aminomethan, Aminocarbonsäuren, insbesondere Glycin oder Histidin, oder Aminoguanidin, wobei unter diesen Ethanolamin, Diethanolamin, Triethanolamin, Amino-1,3-propandiol, Aminoguanidin und Tri(hydroxymethyl)-aminomethan am meisten bevorzugt sind. Weiterhin sind als nukleophile Reagenzien mit mindestens einer funktionellen Gruppe der Struktur I aromatische Amine ausgewählt aus der Gruppe bestehend aus Anilin, Toluidin, Naphthylamin, Benzylamin, Xyliden, Xyloldiaminen, Naphthalendiaminen, Toluoldiaminen, 3,3'-dimethyl-4,4'-diphenyldiamin, Phenylendiaminen, 2,4'-Methylendianilin, 4,4'-Methylendianilin, Sulfonyldianilin, und Dimethylbenzylamin bevorzugt.

Gemäss einer Ausführungsform, bei der das nukleophile Reagenz mindestens eine primäre Aminogruppe der Struktur I aufweist, handelt es sich bei dem nukleophilen Reagenz um ein C₁- bis C₆-Alkylamin, um ein C₁- bis C₆-Alkyldiamin, um ein C₁- bis C₆-Alkyltriamin, um einen C₁- bis C₁₅ Aminoalkohol um ein C₁- bis C₁₅ Aminodiol, oder um eine C₁- bis C₁₅ Aminocarbonsäure.

Gemäss einer weiteren Ausführungsform ist das nukleophile Reagenz eine heterozyklische, ein Stickstoffatom beinhaltende Verbindung, ausgewählt aus der Gruppe umfassend Pyrrol, Pyridin, Chinolin, Indol, Aza-Cyclopentan, Aza-Cyclohexan, Morpholin, Piperidin, Imidazol oder einem Derivat dieser Verbindungen, wobei unter einem Derivat dieser Verbindungen vorzugsweise ein Derivat verstanden ist, bei dem an ein oder mehr Kohlenstoffatomen oder an dem Stickstoffatomen in den vorstehend genannten Verbindungen eine C₁- bis C₃-Alkylgruppe, besonders bevorzugt eine Methyl-Gruppe oder Ethyl-Gruppe anstelle eines Wasserstoffatoms gebunden ist.

Unter den vorstehend genannten nukleophilen Reagenzien sind insbesondere diejenigen bevorzugt, die wasserlöslich sind, insbesondere diejenigen, die in Wasser bei einer Temperatur von 25°C und bei einem pH-Wert von 7 eine Löslichkeit von mindestens 1 g/L, bevorzugt mindestens 10 g/L und besonders bevorzugt mindestens 100 g/L aufweisen.

Das wässrige System, enthaltend das vorstehend beschriebene nukleophile Reagenz kann auf reinem, vorzugsweise entionisiertem Wasser basieren oder aber auch auf anderen, wässrigen Systemen, insbesondere auf Mischungen aus Wasser und organischen Lösungsmitteln wie Alkoholen, insbesondere Mischungen aus Wasser und Ethanol oder Methanol, wobei die Menge an Wasser vorzugsweise mindestens 50 Gew.-%, besonders bevorzugt mindestens 75 Gew.-% und am meisten bevorzugt mindestens 90 Gew.-%, jeweils bezogen auf das Gesamtgewicht aus Wasser und organischem Lösungsmittel, beträgt, physiologischen Kochsalzlösungen, auf Puffern, insbesondere Puffer beinhaltend dem Fachmann bekannt Pufferkomponenten wie beispielsweise TRIS, HEPES, PIPES, CAPS, CHES, AMP, AMPD oder MOPS in einer Menge in einem Bereich von 0,1 bis 1.000 mMol/l, besonders bevorzugt 1 bis 500 mMol/l und am meisten bevorzugt 10 bis 200 mMol/l, wobei gegebenenfalls eine solche Pufferkomponente, je nach deren Struktur, zugleich auch als nukleophiles Reagenz dienen kann. Weiterhin können auch Nährmedien, wie etwa MEM-Medium und DMEM-Medium als wässriges System eingesetzt werden. Die Herstellung der wässrigen Lösung, die das nukleophile Reagenz enthält, erfolgt vorzugsweise durch einfaches Vermischen von Wasser oder einem entsprechenden wässrigen System mit dem nukleophilen Reagenz.

Die Konzentration des nukleophilen Reagenzes in der wässrigen Lösung liegt vorzugsweise in einem Bereich von 0,1 bis 10.000 mMol/l, besonders bevorzugt von 1 bis 5.000 mMol/l, insbesondere bevorzugt von 5 bis 2.500 mMol/l und am meisten bevorzugt von 20 bis 1.000 mMol/l. Gemäss einer besonders vorteilhaften Ausführungsform des erfindungsgemäßen Verfahrens liegt die Konzentration des nukleophilen Reagenzes in der wässrigen Lösung bei mehr als 20 mMol/l, besonders bevorzugt bei mehr als 50 mMol/l und am meisten bevorzugt bei mehr als 100 mMol/l.

Proteolytisch wirksame Verbindungen im Sinne der Erfindung sind alle Protein aufspaltenden Verbindungen, beispielsweise proteolytisch wirksame Enzyme wie Proteasen, insbesondere Proteinase K, Trypsin, Chymotrypsin, Papain, Pepsin, Pronase und Endoproteinase Lys-C zu verstehen. Ferner sind proteolytisch wirksame Verbindungen im Sinne der Erfindung auch nicht-enzymatische Substanzen, die zur Spaltung von Proteinen geeignet sind, wie beispielsweise Bromcyan.

Gemäß Schritt b) des erfindungsgemäßen Verfahrens wird ein Schritt zur Trennung der in dem Ausgangsmaterial befindlichen unterschiedlichen Biomoleküle in wenigstens eine Fraktion (A) und wenigstens eine Fraktion (B) durchgeführt.

Diese Trennung der Fraktionen erfolgt bevorzugt in wenigstens eine lösliche Fraktion (A) und wenigstens eine unlösliche Fraktion (B). Zwar kann auch die gesamte Probe einschließlich der löslichen und unlöslichen Bestandteile in wenigstens zwei Fraktionen aufgeteilt werden, aus denen dann nachfolgend unterschiedliche Biomoleküle isoliert oder aufgereinigt, bzw. in denen unterschiedliche Biomoleküle nachgewiesen oder analysiert werden können, jedoch ist es bevorzugt, dass nach Schritt (a) die Probe in wenigstens eine lösliche Fraktion (A) und wenigstens eine unlösliche Fraktion (B) getrennt wird.

Ein Vorteil dieser bevorzugten Ausführungsformen der Schritte (a) und (b) des erfindungsgemäßen Verfahrens ist, dass die sich in der löslichen Fraktion (A) befindlichen extrahierten/isolierten, weitgehend intakten Proteine in Schritt (c) auch ohne einen weiteren Aufreinigungsschritt analysiert, quantifiziert oder spezifisch fraktioniert werden können. Insbesondere können die extrahierten Proteine mit einem oder mehreren Verfahrensschritten fraktioniert werden. Selbstverständlich können aber auch die in Fraktion (A) befindlichen Proteine gegenüber anderen löslichen Zellbestandteilen oder auch einzelne Proteine gegenüber anderen in Fraktion (A) befindlichen Proteinen aufgereinigt werden, bevor sie nachgewiesen, analysiert oder fraktioniert werden.

Die bevorzugt unlösliche Bestandteile enthaltende Fraktion (B) wird bevorzugt in einem weiteren Schritt d) einer weiteren Behandlung zum Aufschluss der ungelösten Probenbestandteile und gegebenenfalls zum Lösen der Vernetzung von Biomolekülen unterzogen. Die weitgehend noch in der unlöslichen Fraktion (B) befindlichen herauslösbaren Nukleinsäuren können durch diesen weiteren Behandlungsschritt isoliert werden.

Zur Behandlung gemäß Schritt d) ist jedes für das Herauslösen von Nukleinsäuren aus fixiertem Gewebe bekannte Verfahren geeignet, beispielsweise die Verfahren, wie sie in den internationalen Anmeldungen WO2007/068764, WO2008/021419, WO 2005/012523 oder WO2005/054466 beschrieben sind, oder auch die Verfahren, die mit Hilfe von auf dem Markt befindlichen Kits durchgeführt werden können, beispielsweise RNeasy FFPE® und QIAamp FFPE® (beide von Qiagen, Hilden, Deutschland). Bei letzteren werden die unlöslichen Bestandteile der Fraktion (B) wenigstens einem weiteren Hitzeschritt und einer Behandlung mit einer Protease ausgesetzt. Die Protease-Behandlung bewirkt eine effektive Lyse und somit eine Freisetzung der herauslösbaren Nukleinsäuren.

Die Isolierung und ggf. Aufreinigung der Nukleinsäuren erfolgt getrennt von der Isolierung und ggf. Aufreinigung der Proteine, wobei die weitere Bearbeitung der Fraktionen (A) und (B) zeitgleich, aber getrennt voneinander, oder nacheinander erfolgen kann.

Das Aufreinigen/Isolieren der Proteine kann durch chromatographische Methoden, elektrophoretische Trennung, spezifische Bindung an Protein-bindende Materialien, "Protein capturing" mit Hilfe von spezifischen Antikörpern oder durch Fällung erfolgen.

Die Isolierung und Aufreinigung der Nukleinsäuren nach einem weiteren Hitzeschritt und der Protease-Behandlung der Fraktion (B) kann auch durch einfache Fällung, durch Binden der Nukleinsäuren an ein Nukleinsäure-bindendes Material, Elektrophorese oder Chromatographie oder durch ähnliche geeignete und dem Fachmann geläufige Verfahren erfolgen. Bevorzugt werden die Nukleinsäuren mit Hilfe eines Verfahrens isoliert und gereinigt, wie es in WO2007/068764 oder WO2008/021419 beschrieben ist, oder mit Hilfe der RNeasy FFPE® oder QIAamp FFPE® Kits (beide von Qiagen, Hilden, Deutschland) nach den dort vorgegebenen Verfahren.

Die Analyse erfolgt bevorzugt nach einer der oben genannten Analysemethoden. Die Quantifizierung der Proteine erfolgt vorzugsweise mittels der Methode nach Lowry oder BCA, wobei auch andere Quantifizierungsmethoden, insbesondere Proteinarrays, hierfür verwendet werden können. Die Quantifizierung der Nukleinsäuren erfolgt durch jede geeignete Methode, beispielsweise durch quantitative PCR, durch Messung der Optischen Dichte einer definierten Verdünnung bei 260/280 nm oder durch vergleichende Elektrophorese mit vorgegebenen Mengen an Nukleinsäuren.

Die extrahierten Proteine können außerdem mittels proteolytischer Enzyme wie Trypsin, Chymotrypsin, Proteinase K, Papain, Pepsin, Pronase, Endoproteinase LysC, Endoproteinase Glu-C - oder Glykosidasen - wie Endoglycosidase H, NGlycosidase F, Neuroaminidase) oder Phosphatasen behandelt werden.

Vorteilhaft ist, dass die Proteine für mindestens einen biochemischen Assay verwendet werden können. Beispielsweise ist ein bevorzugter biochemischer Assay ein Proteinarray wie ein Mikroarray, insbesondere ein Sandwich Immunoarray, Antigenfängerarray oder ein direkter Proteinarray.

Der biochemische Assay kann vorzugsweise zur Bestimmung von einem oder mehreren diagnostisch oder klinisch relevanten Markerproteinen dienen. Dabei können beispielsweise die Markerproteine aus mindestens zwei biologischen Proben miteinander verglichen werden. Somit kann beispielsweise krank von gesund unterschieden werden. Ferner kann man den Assay auch in einem höheren Multiplexgrad durchführen, um mindestens einen oder mehrere relevante Marker zu analysieren.

Die Nukleinsäuren können in allen bekannten Analyseverfahren, insbesondere in den oben genannten Verfahren ohne Beschränkung eingesetzt werden.

Ebenfalls im Umfang der vorliegenden Erfindung wird ein Kit zur Verfügung gestellt, mit dem sowohl intakte Proteine, als auch Nukleinsäuren aus Formalin fixierten menschlichen oder tierischen biologischen Proben wie Geweben verlässlich und mit hoher Ausbeute isoliert werden können. Bestandteile dieses Kits sind wenigstens (1) ein Protease freies wässriges System zum Lösen der Vernetzungen des Ausgangsmaterials, bevorzugt ein Puffersystem, (2) ein Detergenz (dies kann ggf. in der Lösung (1) vorliegen) und (3) eine Protease haltige Lösung, bzw. eine Protease, wobei erfindungsgemäß das wässrige System (1) ein Puffer ist, der wenigstens ein reduzierendes Reagenz, wenigstens ein Detergenz und wenigstens ein nukleophiles Reagenz umfasst. Weitere Bestandteile des Kits können sein: (4) wenigstens ein Nukleinsäure-bindendes Material, (5) weitere Lösungen oder Puffer zur Nukleinsäureisolierung, bevorzugt ein Bindepuffer und ein Elutionspuffer. Ein detailliertes Protokoll zur Protein- und Nukleinsäureisolierung aus Formalin fixierten Geweben kann dem Kit beigelegt werden.

Das wässrige System (1), das in dem Kit vorliegt, entspricht dem oben beschriebenen wässrigen System mit allen oben genannten möglichen Bestandteilen.

Das nachfolgende Beispiel soll die sich ergebenden Vorteile der vorliegen Methode beschreiben. So zeigte sich z.B., dass das Medikament Iressa® nicht zu einer signifikanten Verbesserung der Überlebensrate von Patienten mit nicht-kleinzelligem Lungenkarzinom (non small cell lung cancer, NSCLC), der häufigsten Lungenkrebsvariante, führte. Insgesamt gesehen, wirkte dieser EGFR-Inhibitor lediglich bei jedem zehnten Patienten. Man fand Hinweise auf antitumorigene Wirksamkeit bei Frauen, Nichtrauchern und Patienten asiatischer Herkunft. Die Gründe hierfür waren, dass diese Patienten Genmutationen in dem EGFR-Gen trugen, die zur Sensitivität gegenüber Iressa® führten. Die Überexpression des Rezeptors spielte hingegen eine untergeordnete Rolle, war aber das ausschlaggebende Kriterium während der Entwicklung des neuen Medikaments. Mit einem geeigneten Test könnten sowohl diese Mutationen als auch die Expression aus derselben Probe ohne wesentliche Verluste analysiert werden und somit festgestellt werden, welcher Patient auf eine Behandlung mit Iressa® reagieren sollte. Eine größere Probenentnahme oder eine Entnahme von zwei Proben für die jeweils separate Isolierung von DNA und Proteinen wäre somit vermeidbar.

Da es in Zukunft mehr und mehr zielgerichtete Therapien geben wird und die entsprechende Diagnostik dazu parallel entwickelt werden muss, kommt der parallelen Analyse von Nukleinsäuren (insbesondere DNA, RNA) und Proteinen aus Extrakten von FFPE Geweben eine Schlüsselrolle für die prädiktive Medizin zu. Hierbei ist es unerlässlich, diese zu quantifizieren, d.h. das Expressionsniveau oder die Translationsrate oder auch beide Parameter einzelner Komponenten zu ermitteln, und diese auch auf Veränderungen hinsichtlich der Gensequenz (Mutationen, Deletionen) zu untersuchen.

### Beispiele

### Beispiel 1: Trennung der Biomoleküle durch das erfindungsgemäße Verfahren.

Für diesen Versuch wurden in Formalin-fixierte und in Paraffin eingebettete Gewebeproben (FFPE-Proben) aus Ratten-Leber verwendet. Von diesen Proben wurden mit Hilfe eines Microtoms Schnitte von ca. 10µm Dicke hergestellt und 2 Schnitte je Probe eingesetzt. Für die folgende Isolierung von Proteinen und DNA/RNA aus den FFPE-Schnitten wurden Komponenten des RNeasy FFPE Kits, des QIAamp FFPE Kits, des Qproteome FFPE Kits und des Allprep DNA/RNA Mini Kits des Herstellers QIAGEN verwendet.

Die Gewebe wurden zunächst nach üblichen Protokollen entparaffiniert. Z. B. wurden die Proben zunächst 10 min in Xylol inkubiert. Nach Pelletierung der Probe und Abnehmen des Überstandes wurde diese Behandlung mit Xylol zwei weitere Male durchgeführt. Im Anschluß wurden die Proben je 2 mal mit 100% Ethanol, mit 96% Ethanol und 70% Ethanol wie oben beschrieben behandelt.

Die so entstandenen, entparaffinierten Probenpellets wurden zur Freisetzung der Volllängen-Proteine mit 100µl EXB-Puffer der Firma QIAGEN versetzt und für 20 min bei 100°C gekocht. Anschließend wurden die Proben für weitere 2 Stunden bei 80°C inkubiert. Auf diese Weise wurden die Proteine aus der Probe freigesetzt und die durch das Formalin hervorgerufenen Quervernetzungen gelöst. Durch diese erfindungsgemäße Behandlung der Proben wurden die Nukleinsäuren nicht bzw. nur in unwesentlichem Maße in Lösung freigesetzt und konnten selektiv durch eine Zentrifugation, z.B. für 15 min bei 14000xg pelletiert werden, während die Proteine löslich im Überstand verblieben, der durch Abnehmen vom Pellet getrennt wurde. Neben den sedimentierten Nukleinsäuren wurden auch völlig unlösliche Bestandteile zusammen mit den Nukleinsäuren abgetrennt, die aber keinen Einfluss auf die weitere Aufreinigung von DNA und RNA hatten.

Um den Verbleib der verschiedenen Biomoleküle, Proteine und Nukleinsäuren, in den beiden Fraktionen zu klären wurde zunächst, nach der Zentrifugation der behandelten Proben, der Überstand abgenommen und getrennt vom Pellet für weitere Analysen und Aufarbeitungen verwendet. Um die Verteilung der Biomoleküle auf die Fraktionen zu untersuchen wurden 3 Proben verwendet. Von Probe 1 wurden die Proteine aus Überstand und Pellet isoliert, von Probe 2 wurde DNA aus Überstand und Pellet isoliert und Probe 3 diente zu Isolierung von RNA aus Überstand und Pellet.

Der Überstand von Probe 1 wurde ohne weitere Bearbeitung zur Analyse der enthaltenen Proteine eingesetzt.

Für die DNA-Isolierung aus der 2. Probe wurde der Überstand mit 200µl eines chaotropen Lysepuffers, z. B. dem Puffer RBC der Firma QIAGEN, versetzt und das Gemisch auf eine Silica-Membran, z. B. in der Allprep DNA-Säule der Firma QIAGEN, aufgetragen und durch Zentrifugation für 1 min bei 10000rpm durch die Membran geführt. Durch Zugabe des chaotropen Reagenzes werden Bedingungen geschaffen, welche die Bindung der DNA, aber nicht der RNA, an die Silicamembran ermöglichen. Die Silicamembran wurde dann durch die Durchführung von 500µl des Guanidinsalz-haltigen Waschpuffers AW1 und anschließend von 500µl des dem alkoholhaltigen Waschpuffers AW2 und von 500µl 100% Ethanol gewaschen. Die Membran wurde durch eine 5 minütige Zentrifugation bei 14000rpm getrocknet. Die DNA wurde durch Auftrag von 30µl eines DNA-Elutionspuffers, z. B: ATE der Firma QIAGEN, nach einminütiger Inkubation durch Zentrifugation eluiert.

Analog wurde für die RNA-Isolierung aus der dritten Probe der Überstand mit 200µl eines chaotropen Lysepuffers, z. B. dem Puffer RBC der Firma QIAGEN, versetzt und das Gemisch auf eine Silica-Membran, z. B. in der Allprep DNA-Säule der Firma QIAGEN, aufgetragen und durch Zentrifugation für 1 min bei 10000rpm durch die Membran geführt. Da die Zusammensetzung des Gemisches zur Bindung der DNA, aber nicht der RNA an die Silicamembran führt, befindet sich die RNA im Durchfluß der Säule. Dieser Durchfluß wurde zum Einstellen von Bindebedingungen für RNA mit Ethanol vermischt und dann erneut auf eine Silicamembran, z. B. in der RNeasy MinElute Säule der Firma QIAGEN, aufgetragen und durch Zentrifugation für 1 min bei 10000rpm durch die Membran geführt. Die Silicamembran wurde dann durch die Durchführung von 500µl des Guanidinsalz-haltigen Waschpuffers RW1 und anschließend von 500µl des dem alkoholhaltigen Waschpuffers RW2 gewaschen. Die Membran wurde durch eine 5-minütige Zentrifugation bei 14000rpm getrocknet. Die RNA wurde durch Auftrag von 30µl Wasser nach einminütiger Inkubation durch Zentrifugation eluiert.

Auch die Pelletfraktionen der drei Proben, welche durch Zentrifugation der erfindungsgemäß behandelten Probe erhalten wurden, wurden für die Isolierung von Proteinen (Probe 1), DNA (Probe 2) und RNA (Probe 3) verwendet.
Zur Isolierung der Proteine wurde das Pellet von Probe 1 in einem geeigeneten Proteinlysepuffer, wie z. B. dem detergenzhaltigen Mammalian Protein Lysis Puffer, gelöst und zur weiteren Analyse eingesetzt.

Zur DNA-Isolierung wurde das Pellet von Probe 2 mit einem üblichen DNA-Lysepuffer, wie z. B. 180µl des detergenzhaltigen Puffers ATL des Herstellers QIAGEN, versetzt. Da das Pellet nur Bestandteile aufwies, die durch die vorhergehenede Behandlung noch nicht gelöst worden waren, erfolgte außerdem eine Lyse mittels einer Protease, z. B. 20µl Proteinase K, um diese ungelösten Bestandteile ebenfalls in Lösung zu bringen. Nach einer einstündigen Inkubation bei 56°C und anschließender 10 minütiger Inkubation bei 90°C, wurde das Lysat mit einem chaotrophaltigen Bindepuffer, z. B. dem Puffer AL des Herstellers QIAGEN versetzt und das Gemisch auf eine Silica-Membran, z. B. in der QIAamp Mini Säule der Firma QIAGEN, aufgetragen und durch Zentrifugation für 1 min bei 10000rpm durch die Membran geführt. Die Silicamembran wurde wie oben beschrieben durch die Durchführung von Waschpuffern AW1, AW2 und 100% Ethanol, gewaschen, die Membran getrocknet und die DNA eluiert.

Zur RNA-Isolierung wurde das Pellet von Probe 3 mit einem üblichen RNA-Lysepuffer, wie z. B. 150µl des detergenzhaltigen Puffers PKD und 10µl Proteinase K des Herstellers QIAGEN, versetzt. Nach einer einstündigen Inkubation bei 56°C und anschließender 15 minütiger Inkubation bei 80°C, wurde das Lysat mit einem chaotrophaltigen Bindepuffer, z. B. dem Puffer RBC des Herstellers QIAGEN versetzt und das Gemisch auf eine Silica-Membran, z. B. in der RNeasy MinElute Säule der Firma QIAGEN, aufgetragen und durch Zentrifugation für 1 min bei 10000rpm durch die Membran geführt. Die Silicamembran wurde wie oben beschrieben durch die Durchführung von Waschpuffern, RW1 und RW2 gewaschen, die Membran getrocknet und die RNA eluiert.

Um die Verteilung der so aus den beiden Fraktionen Überstand und Pellet isolierten Proteine und Nukleinsäuren zu bestimmen, wurden die Biomoleküle aus beiden Fraktionen mit geeigneten Methoden quantifiziert. Zur Bestimmung der Ausbeute der enthaltenen Proteine wurde ein BCA-Test nach Angaben des Herstellers (Pierce) eingesetzt. Die Bestimmung der Ausbeute und Reinheit der DNA- bzw. RNA erfolgte durch Messung der Absorption bei 260/280nm. Die Mittelwerte der Ausbeuten der Mehrfachbestimmungen sind in Tabelle 1 dargestellt.

**Tabelle 1:**

| Analyt | Fraktion | |
|---|---|---|
| | Überstand Ausbeute/µg | Pellet Ausbeute/µg |
| Protein (Probe 1) | 23,87 | 5,56 |
| RNA (Probe 3) | 3,94 | 16,74 |
| DNA (Probe 2) | 1,04 | 4,53 |

Die Ergebnisse zeigen, dass bei Anwendung des erfindungsgemäßen Verfahrens eine deutliche Fraktionierung der Biomoleküle vor der nachgeschaltenen Aufreinigung erfolgt. Die Proteine befanden sich absolut überwiegend im Überstand, während die Nukleinsäuren in der Pelletfraktion lokalisiert waren.

### Beispiel 2: Gelanalyse der nach dem erfindungsgemäßen Verfahren fraktionierten Biomoleküle

DNA, RNA und Proteine, welche nach den in Beispiel 1 beschriebenen Verfahren aus den Pelletfraktionen und den Überstandfraktionen isoliert wurden, sind mittels Gelanalysen weiter analysiert worden.

Gleiche Volumen der aus den beiden Fraktionen isolierten Proteine wurden nach üblichen Methoden mittels eines SDS-Polyacrylamid-Gels, welches anschließend mit Coomassie angefärbt wurde, analysiert. Hier waren ausschließlich in der Überstandfraktion Proteine sichtbar, während die Pelletfraktionen keinerlei sichtbare Proteine aufwies.

Gleiche Volumen der aus den beiden Fraktionen isolierten Nukleinsäuren wurden nach üblichen Methoden auf einem TAE-Agarosegel aufgetrennt und mit Ethidiumbromid angefärbt. Das Ergebnis ist in Abbildung 1 gezeigt. Die mit Ethidiumbromid angefärbten Gele zeigten in allen Fällen in den Fraktionen sowie in den Kontrollen fragmentierte DNA bzw. RNA, erkennbar an einem hellen "Schmier" verteilt über einen bestimmten Größenbereich. Die aus FFPE-Proben isolierbaren Nukleinsäuren waren immer fragmentiert, da die Nukleinsäuren bereits während der Fixierung, Einbettung und Lagerung der Proben degradiert wurden. Es ist außerdem deutlich zu erkennen, dass die Pelletfraktion sowohl deutlich mehr DNA, als auch RNA, als die jeweils zugehörige Überstandfraktion aufwies.

### Beispiel 3: Realtime RT-PCR Analyse der nach dem erfindungsgemäßen Verfahren fraktionierten RNA

Um die Wirkung des erfindungsgemäßen Verfahrens nicht nur auf die Isolierung der Nukleinsäuren, sondern auch auf die Analyse mittels Amplifikation zu untersuchen, wurde die RNA, welche nach den in Beispiel 1 beschriebenen Verfahren aus der Pelletfraktion und der Überstandfraktion isoliert wurde, zur weiteren Analyse mittels quantitativer Real-Time-RT-PCR eingesetzt.

Die isolierte RNA wurde jeweils in Doppelbestimmung für den Nachweis eines Amplicons des madH7 Transkriptes verwendet. Die Eluate wurden jeweils 1:5 mit Wasser verdünnt und 5 µl dieser Verdünnungen in die Realtime-PCR eingesetzt. Die Amplifikation erfolgte in einem Gesamtvolumen von 25µl mit einem geeigneten Mastermix zur Real-Time-RT-PCR, wie z.B. dem QuantiTect SYBRGreen RT-PCR-Kit der Firma QIAGEN, laut Angaben des Herstellers. Die Amplifikation erfolgte in einem geeigneten Real-Time-Amplifikationsgerät, wie z. B. dem 7700 der Firma ABI. Aus den ermittelten ct-Werten wurden die Mittelwerte über die Doppelbestimmungen der Duplikate und die Standartabweichung ermittelt. Das Ergebnis ist in Tabelle 2 dargestellt.

**Tabelle 2:**

| Fraktion | Mittelwert ct | Standardabweichung |
|---|---|---|
| Pellet | 24,7 | 0,26 |
| Überstand | 28,1 | 0,25 |

Die Ergebnisse zeigen, dass die nach dem erfindungsgemäßen Verfahren isolierte RNA, insbesondere aus der Pelletfraktion, gut für Amplifikationsanalysen geeignet war. Weiterhin bestätigen sie, dass der überwiegende Anteil der RNA in der Pelletfraktion lokalisiert war, während nur ein deutlich geringerer Anteil im Überstand nachzuweisen war.

Zusammengefasst bestätigen sowohl die Ausbeutemessungen als auch die weiteren Analysen von Proteinen und Nukleinsäuren, dass bei Anwendung des erfindungsgemäßen Verfahrens eine deutliche Fraktionierung der Biomoleküle vor der nachgeschaltenen Aufreinigung erfolgt. Die Proteine befinden sich absolut überwiegend im Überstand, während die Nukleinsäuren in der Pelletfraktion lokalisiert sind.

## Patentansprüche

1. Verfahren zur parallelen Aufreinigung verschiedener Arten von Biomolekülen aus demselben durch Vernetzung fixierten biologischen Ausgangsmaterial, wobei
a) ein Schritt zum Lösen der Vernetzungen des Ausgangsmaterials unter Verwendung eines Puffer, welcher ein Detergenz und keine proteolytisch wirksame Verbindung enthält
b) ein Schritt zur Trennung der in dem Ausgangsmaterial befindlichen unterschiedlichen Biomoleküle in wenigstens eine lösliche Fraktion (A), die weitgehend intakte Proteine enthält, und wenigstens eine unlösliche Fraktion (B) und
c) Isolierung oder Nachweis/Analyse oder Isolierung und Nachweis/Analyse unterschiedlicher Biomoleküle aus den Fraktionen (A) und (B) aus Schritt b)
erfolgt.

2. Verfahren nach Anspruch 1, wobei in Schritt a) die Vernetzung des Ausgangsmaterials durch folgende Schritte gelöst wird:
(i) Überführen des Materials in einen Puffer, welcher ein Detergenz und keine proteolytisch wirksame Verbindung enthält,
(ii) Inkubieren des Materials in dem Puffer bei einer Temperatur, zwischen 95 und 100°C,
anschließend weitere Inkubation bei einer Temperatur oberhalb von 60°C.

3. Verfahren nach einem der Ansprüche 1 oder 2, wobei durch das Trennen der Fraktion (A) von der Fraktion (B) wenigstens die aus dem Ausgangmaterial herauslösbaren Proteine weitgehend quantitativ von wenigstens den herauslösbaren Nukleinsäuren getrennt werden, wobei sich die lösbaren Proteine weitgehend quantitativ in Fraktion (A) befinden, wogegen sich die lösbaren Nukleinsäuren weitgehend quantitativ in Fraktion (B) befinden.

4. Verfahren nach einem der Ansprüche 1 bis 3, wobei die Isolierung der unterschiedlichen Biomoleküle gemäß Schritt c) durch Aufreinigen/Isolieren von Proteinen aus Fraktion (A) mit Hilfe üblicher Methoden zur Proteinaufreinigung oder Proteinisolierung erfolgt und die Isolierung von Nukleinsäuren aus Fraktion (B) durch einen weiteren Schritt d) erfolgt, der wenigstens eine weitere Behandlung zum Aufschluß der ungelösten Probenbestandteile und/oder ein weiteres Lösen noch bestehender Vernetzungen in dem Ausgangsmaterial umfasst, sowie das Isolieren der lösbaren Nukleinsäuren.

5. Verfahren nach einem der Ansprüche 1 bis 4, wobei das Aufreinigen / Isolieren der Proteine durch chromatographische Methoden, elektrophoretische Trennung, spezifische Bindung an Protein-bindende Materialien, "Protein capturing" mit Hilfe von spezifischen Antikörpern oder durch Fällung erfolgt und das Isolieren der Nukleinsäuren durch Fällung, Bindung an Nukleinsäure-bindende Materialien, Elektrophorese oder Chromatographie erfolgt.

6. Verfahren nach einem der Ansprüche 1 bis 5, wobei der Nachweis/die Analyse von Proteinen durch immunologische Methoden, chromatographische Methoden Sequenzierung oder Elektrophorese, der Nachweis/die Analyse der Nukleinsäuren durch spezifische PCR, Elektrophorese, Hybridisierungsverfahren oder Sequenzierung erfolgt.

7. Kit zur Durchführung eines Verfahrens gemäß einem der Ansprüche 1 bis 6, enthaltend wenigstens (1) ein Protease freies wässriges System zum Lösen der Vernetzungen des Ausgangsmaterials, bevorzugt ein Puffersystem, (2) ein Detergenz welches ggf. in der Lösung (1) vorliegen kann und (3) eine Protease haltige Lösung, bzw eine Protease, **dadurch gekennzeichnet, dass** das wässrige System (1) ein Puffer ist, der wenigstens ein reduzierendes Reagenz, wenigstens ein Detergenz und wenigstens ein nukleophiles Reagenz umfasst.

8. Kit gemäß Anspruch 7, zusätzlich enthaltend (4) wenigstens ein Nukleinsäure-bindendes Material, (5) weitere Puffer zur Nukleinsäureisolierung, bevorzugt eine Bindepuffer und ein Elutionspuffer.

9. Verwendung des Kits gemäß einem der Ansprüche 7 oder 8 für die Analyse und/oder Quantifizierung von in biologischen Proben enthaltenen Biomolekülen.

10. Verwendung des Kits gemäß einem der Ansprüche 7 oder 8 für die Diagnose, Prognose, Therapieentscheidung und das Therapiemonitoring einer Krankheit anhand von Proben außerhalb eines menschlichen oder tierischen Körpers.

## Claims

1. Method for the parallel purification of various types of biomolecules from the same biological base material fixed by cross-linking, wherein
a) a step for dissolving the cross-linking of the base material, using a buffer containing a detergent and no proteolytically acting compound,
b) a step for separating the different biomolecules present in the base material into at least one soluble fraction (A) containing the mostly intact proteins, and at least one insoluble fraction (B), and
c) isolation or confirmation/analysis or isolation and confirmation/analysis of different biomolecules from fractions (A) and (B) from step b)
take place.

2. Method according to claim 1, wherein the cross-linking of the base material is dissolved with the following steps during step a):
(i) Transfer of the material into a buffer containing a detergent and no proteolytically acting compound,
(ii) incubation of the material in the buffer at a temperature between 95 and 100°C, subsequent further incubation at a temperature above 60°C.

3. Method according to one of the claims 1 or 2, wherein at least the proteins separatable from the base material by separating fraction (A) from fraction (B) are mostly quantitatively separated from at least the separatable nucleic acid, wherein the dissolvable proteins are mostly quantitatively present in fraction (A), whilst the soluble nucleic acids are mostly quantitatively present in fraction (B).

4. Method according to one of the claims 1 to 3, wherein the isolation of the various biomolecules according to step c) is carried out by purification/isolation of proteins from fraction (A) with the help of usual methods for protein purification or protein isolation, and the isolation of nucleic acids from fraction (B) is carried out by means of a further step d), which comprises at least one further treatment for decomposing the undissolved sample components and/or a further dissolving of cross-linking still present in the base material, and the isolating of dissolvable nucleic acids.

5. Method according to one of the claims 1 to 4, wherein the purification/isolation of the proteins takes place by means of chromatographic methods, electrophoretic separation, specific binding to protein binding materials, "protein capturing" with the aid of specific antibodies or through precipitation, and the isolating of nucleic acids takes place by means of precipitation, binding to nucleic acid binding materials, electrophoresis or chromatography.

6. Method according to one of the claims 1 to 5, wherein the confirmation/analysis of proteins takes place by means of immunological methods, chromatographic methods, sequencing or electrophoresis, the confirmation/analysis of nucleic acids takes place by means of specific PCR, electrophoresis, hybridisation methods or sequencing.

7. Kit for carrying out a method according to one of the claims 1 to 6, containing at least (1) a protease-free aqueous system for dissolving cross-linking in the base material, preferably a buffer system, (2) a detergent that can be present in the solution (1), and (3) a protease containing solution, or a protease, **characterised in that** the aqueous system (1) is a buffer that comprises at least one reducing reagent, at least one detergent and at least one nucleophilic reagent.

8. Kit according to claim 7, additionally containing (4) at least one nucleic acid binding material, (5) further buffers for nucleic acid isolation, preferably a binding buffer, and an elution buffer.

9. Use of the kit according to one of the claims 7 or 8 for the analysis and/or quantification of biomolecules contained in biological samples.

10. Use of the kit according to one of the claims 7 or 8 for the diagnosis, prognosis, treatment decision and treatment monitoring of a medical condition by means of samples outside of a human or animal body.

## Revendications

1. Procédé de purification en parallèle de biomolécules de différentes sortes, à partir du même matériel biologique de départ fixé par réticulation, comportant
a) une étape de rupture des liaisons de réticulation au sein du matériel de départ, réalisée au moyen d'un tampon qui contient un détergent et ne contient aucun composé doté d'une activité protéolytique,
b) une étape de séparation des différentes biomolécules présentes dans le matériel de départ, en au moins une fraction soluble (A), qui contient des protéines largement intactes, et en au moins une fraction insoluble (B),
c) et l'isolement, ou la détection/analyse, ou l'isolement et la détection/analyse, des différentes biomolécules, à partir des fractions (A) et (B) issues de l'étape (b).

2. Procédé conforme à la revendication 1, dans lequel, dans l'étape (a), on rompt les liaisons de réticulation au sein du matériel de départ en effectuant les opérations suivantes :
i) transfert du matériel dans un tampon qui contient un détergent et ne contient aucun composé doté d'une activité protéolytique,
ii) et incubation du matériel, au sein du tampon, à une température située entre 95 et 100 °C,
suivies d'une incubation supplémentaire à une température supérieure à 60 °C.

3. Procédé conforme à l'une des revendications 1 et 2, dans lequel, en séparant la fraction (A) d'avec la fraction (B), on sépare au moins les protéines libérables du matériel de départ, de manière largement quantitative, d'avec au moins les acides nucléiques libérables, les protéines libérables se trouvant, de manière largement quantitative, dans la fraction (A), mais les acides nucléiques libérables se trouvant par contre, de manière largement quantitative, dans la fraction (B).

4. Procédé conforme à l'une des revendications 1 à 3, dans lequel on opère l'isolement des différentes biomolécules, dans l'étape (c), en effectuant une purification/isolement des protéines issues de la fraction (A) selon les procédés habituels de purification de protéines ou d'isolement de protéines, et l'on réalise l'isolement des acides nucléiques issus de la fraction (B) en effectuant une étape supplémentaire (d) qui comporte au moins un traitement supplémentaire visant à désagréger les constituants non-dissous de l'échantillon et/ou une opération supplémentaire de rupture des liaisons de réticulation encore présentes dans le matériel de départ, ainsi que l'isolement des acides nucléiques libérables.

5. Procédé conforme à l'une des revendications 1 à 4, dans lequel on effectue la purification/isolement des protéines par des méthodes de chromatographie, par séparation par électrophorèse, par liaison spécifique à des substances se liant aux protéines, par capture de protéines à l'aide d'anticorps spécifiques, ou par précipitation, et l'on effectue l'isolement des acides nucléiques par précipitation, par liaison à des substances se liant aux acides nucléiques, par électrophorèse ou par chromatographie.

6. Procédé conforme à l'une des revendications 1 à 5, dans lequel on opère la détection/analyse des protéines par des méthodes immunologiques, par des méthodes de chromatographie, par séquençage ou par électrophorèse, et la détection/analyse des acides nucléiques par des procédés spécifiques de PCR, d'électrophorèse ou d'hybridation ou par séquençage.

7. Trousse pour la mise en oeuvre d'un procédé conforme à l'une des revendications 1 à 6, qui comporte au moins :
1) un système aqueux sans protéases, pour rompre les liaisons de réticulation au sein du matériel de départ, de préférence un système tampon,
2) un détergent, qui peut, en option, se trouver présent dans la solution (1),
3) et une protéase, ou une solution contenant une protéase, **caractérisée en ce que** le système aqueux (1) est un tampon qui comprend au moins un réactif réducteur, au moins un détergent et au moins un réactif nucléophile.

8. Trousse conforme à la revendication 7, qui comporte en outre
4) au moins une substance se liant aux acides nucléiques,
5) et d'autres tampons pour l'isolement des acides nucléiques,
de préférence un tampon de liaison et un tampon d'élution.

9. Utilisation d'une trousse conforme à la revendication 7 ou 8 pour l'analyse et/ou le dosage de biomolécules contenues dans des échantillons biologiques.

10. Utilisation d'une trousse conforme à la revendication 7 ou 8 pour le diagnostic, le pronostic, la décision de traitement ou la surveillance du traitement d'une maladie, à l'aide d'échantillons et hors d'un corps humain ou animal.
